(19) Europäisches Patentamt
European Patent Office
Office européen des brevets

(11) **EP 2 954 321 B1**

(12) **EUROPÄISCHE PATENTSCHRIFT**

(45) Veröffentlichungstag und Bekanntmachung des Hinweises auf die Patenterteilung:
**06.12.2017 Patentblatt 2017/49**

(21) Anmeldenummer: **14703352.6**

(22) Anmeldetag: **07.02.2014**

(51) Int Cl.:
*G01N 33/566* *(2006.01)*    *G01N 33/68* *(2006.01)*
*C07K 14/47* *(2006.01)*

(86) Internationale Anmeldenummer:
**PCT/EP2014/052392**

(87) Internationale Veröffentlichungsnummer:
**WO 2014/122245 (14.08.2014 Gazette 2014/33)**

(54) **METHODE ZUR INHIBIERUNG DES PROTEINS SWAP-70**

METHOD FOR THE INHIBITION OF SWAP-70 PROTEIN

MÉTHODE POUR L'INHIBITION DE LA PROTÉINE DE SWAP-70

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.02.2013 DE 102013202206**

(43) Veröffentlichungstag der Anmeldung:
**16.12.2015 Patentblatt 2015/51**

(73) Patentinhaber: **THORNE LIMITED**
**Vancouver, V6Z1S4 (CA)**

(72) Erfinder:
• **JESSBERGER, Rolf**
**01326 Dresden (DE)**
• **CHACÓN-MARTINEZ, Carlos Andres**
**50935 Köln (DE)**

(74) Vertreter: **Kailuweit & Uhlemann Patentanwälte Partnerschaft mbB**
**Bamberger Straße 49**
**01187 Dresden (DE)**

(56) Entgegenhaltungen:
WO-A2-03/074652       DE-T2- 60 023 261
US-A1- 2009 069 420   US-B1- 6 528 284

• THOMAS WEIDEMANN ET AL: "Analysis of Ligand Binding by Two-Colour Fluorescence Cross-Correlation Spectroscopy", SINGLE MOLECULES, vol. 3, no. 1, 11 March 2002 (2002-03-11), pages 49-61, XP055345461, DE ISSN: 1438-5163, DOI: 10.1002/1438-5171(200204)3:1<49::AID-SIMO49>3.3.CO;2-K

## Beschreibung

Die Erfindung betrifft das Gebiet der biomedizinischen und pharmakologischen Forschung, insbesondere im Bereich Immunologie, Allergien, Krebserkrankungen, Knochenerkrankungen und Autoimmunkrankheiten.

STAND DER TECHNIK

Krebs, Allergien, darunter auch Asthma, Autoimmunkrankheiten, und Osteoporose sind weltweit sehr verbreitete, zum Teil zunehmende Erkrankungen, wobei Krebs, Autoimmunkrankheiten und Allergien in schweren Fällen bis zum Tod führen können.

Die Inzidenz an Allergien hat sich in den letzten 20 Jahren verdreifacht. Zwischen 5 und 10 % der Weltbevölkerung leiden an Allergien, inklusive Asthma, an dem etwa ein Fünftel dieser Personen leiden. Asthma ist die häufigste chronische Erkrankung bei unter 18-Jährigen. Allergien werden durch übermäßige IgE-Produktion der B-Lymphozyten ausgelöst. Eingriffe in die Regulation dieser IgE-Produktion sind somit prinzipiell wegweisend für eine ursächliche Allergietherapie, die bislang nicht existiert. Jedoch ist bisher zu wenig bekannt, wie die IgE-Produktion im Einzelnen reguliert wird.

Die IgE-Produktion hängt, wie seit längerem bekannt, von der die Transkription aktivierenden Funktion des Proteins STAT-6 ab. Es ist auch bekannt, dass der Effekt von STAT-6 auf die IgE-Produktion durch BCL-6 inhibiert wird. BCL-6 und STAT-6 binden einen überlappenden Bereich der regulatorischen DNA-Sequenz des IgE-Gens. Es wird angenommen, dass BCL-6 mit Co-Repressoren interagiert und dadurch aktiv die IgE-Produktion hemmt. Diese Repression ist spezifisch für das IgE-Gen, da BCL-6 selektiv an die STAT-6-Bindungstelle im IgE-Gen bindet und an die meisten anderen STAT-6-Bindungsstellen nicht bindet. Die exakten Mechanismen der Spezifität und der Regulation der kritischen Balance zwischen BCL-6 und STAT-6 sind jedoch nicht bekannt.

Das SWAP-70 Protein wird hauptsächlich in aktivierten B-Zellen, in Mastzellen, in Osteoklasten und in dendritischen Zellen exprimiert, obwohl die Präsenz des Proteins im Zytoplasma anderer Zelltypen nicht ausgeschlossen werden kann (Borggrefe et al.1998; Borggrefe et al. 1999; Masat et al. 2000; Gross et al, 2002; Pearce et al., 2006; Ocana-Morgner et al., 2009). Insbesondere bei Zelltransformation, d.h. der Entstehung von Tumorzellen aus primären Zellen, und in Tumorzelllinien verschiedenen Ursprungs wird SWAP-70 exprimiert. Es ist bekannt, dass SWAP-70 defiziente Mäuse spezielle Veränderungen des Phenotyps aufweisen, u. a. sind die CD40-vermittelte Aktivierung von B-Zellen einschließlich dem Wechsel zur Produktion von IgE, und die Degranulierung von Mastzellen stark reduziert (Borggrefe et al., 2001; Gross et al., 2002). Hinsichtlich der IgE-Produktion in Abhängigkeit von SWAP-70 wurde gezeigt, dass in SWAP-70 defizienten Mäusen vor und nach Immunisierung stark verminderte IgE-Konzentrationen vorliegen, und dass die Produktion sekretierten IgEs in B-Zell-Kulturen um ca. das 8-fache reduziert ist. Die Produktion anderer Immunoglobulin-Klassen ist jedoch nicht wesentlich beeinträchtigt.

In US 6,528,284 B1 (US 2003/143611 A1, WO 99/03991 A1) wird auf die Reinigung und Charakterisierung von Proteinen, die generell am Immunoglobulin-Klassenwechsel beteiligt sind eingegangen. Identifiziert wurde SWAP-70, welches hier als SRTA-70 (S-Region Transfer Activity-70) bezeichnet und beansprucht wird. Beschrieben wird dessen allgemeine Rolle im Immunoglobulin-Klassenwechsel, sowie die Nutzung dieses Proteins in der Modulation des Klassenwechsels.

US 2003/0166018 A1 (WO 03/074652 A2) beschreibt die Rolle von SWAP-70 in der Mastzelldegranulation und beansprucht eine Methode zur Identifizierung eines Agens, welches den Gehalt von SWAP-70 Protein in einer Mastzelle reduziert.

WO 2011/141267 A1 offenbart ein Screening-Verfahren zur Identifizierung von Wirkstoffen, die die IgE-Produktion hemmen. Basis dieses Verfahrens ist die regulatorische Wirkung von SWAP-70 auf STAT-6 und BCL-6, welche wiederum die IgE-Produktion beeinflussen.

Die Allergiebehandlung erfolgt heutzutage überwiegend mit Hilfe von Symptombehandlung, d.h. Linderung der allergischen Reaktion beispielsweise durch anti-Histaminika oder Glucocorticoide, nicht aber durch ursächliche Therapie. Nachteile bisheriger Therapien sind u. a. kurze Wirkung, keine Ursachenbehebung, unspezifische Wirkung und Nebenwirkungen.

Der einzige bisher bekannte IgE-Hemmer ist Omalizumab, ein rekombinanter humanisierter monoklonaler Antikörper gegen Immunglobulin E (Anti-IgE) zur Behandlung von schwerem allergischen Asthma bronchiale. Aber auch Omalizumab unterbindet nicht ursächlich die IgE-Produktion, sondern fängt vielmehr bereits produziertes IgE ab, wodurch Immunglobulinkomplexe entstehen. Nachteilig können Antikörpertherapien selbst allergische Reaktionen bzw. das Antikörperprotein attackierende Immunreaktionen auslösen. Zudem sind Antikörper pharmakologisch aufwändig herzustellen.

Des Weiteren spielt SWAP-70 eine wichtige Rolle bei Krebserkrankungen. Es wurde gezeigt, dass Überexpression von SWAP-70 humane Zellen zu Tumorzellen transformieren kann. Ferner wurde gezeigt, dass SWAP-70 die Zellwanderung von Tumorzellen unterstützt, d.h. deren Invasivität fördert, was insbesondere bei Metastasenbildung von Bedeutung ist. In verschiedenen Tumorzellen bzw. menschlichen Tumoren tritt SWAP-70 überexprimiert und/oder mutiert auf. Beispielsweise werden primäre embryonale Fibroblasten der Maus durch Expression eines mutierten Swap-70 Gens zu Tumorzellen transformiert.

Zudem konnte gezeigt werden, dass die Aktivität von Osteoklasten, d.h. Knochensubstanzabbauenden Zellen, durch Eliminierung von SWAP-70 gehemmt wird. SWAP-70 unterstützt Osteoklastenaktivität durch Förderung der Wanderung und cytoskeletalen Dynamik dieser Zellen. Bei der Osteoporose sind Osteoklasten hyperaktiv. Hemmung von SWAP-70 wird daher den Knochenabbau bei Osteoporose hemmen.

Allergiebehandlung erfolgt überwiegend mit Hilfe von Symptombehandlung, d.h. Linderung der allergischen Reaktion beispielsweise durch anti-Histaminika mit bekannten Nebenwirkungen, nicht aber durch ursächliche Therapie. Blockierung der IgE-Produktion in B-Zellen und/oder der Mastzelldegranulation stellen ursächliche Therapieansätze dar, die sich auch ergänzen können. Krebstherapie bedarf einer Vielzahl von sich ergänzenden Ansätzen. Der therapeutische Erfolg, sowohl bei Primärtumoren als auch bei Metastasen, ist nach wie vor sehr unbefriedigend. Daher sind neue Ansätze, neue Zielmoleküle, unverzichtbar. Osteoporose, wie die vorgenannten Erkrankungen eine sehr weit verbreitete "Volkskrankheit", wird überwiegend entweder mittels seit längerem eingesetzter Bisphosphonate oder, seit neuerer Zeit (experimentell), mittels Hemmern von Osteoklastenwachstumsfaktoren therapiert. Beide Ansätze haben erhebliche Nachteile. Bisphosphonate hemmen RhoGTPasen in fast allen Zellen sind daher bei längerer Anwendung mit deutlichen Nebenwirkungen behaftet. Wachstumsfaktorhemmer sind oft schwierig herzustellen und/oder sehr teuer (Antikörper, Cytokine) und/oder wirken auf andere Zellen bzw. Systeme (Inflammation).

Auch bei Autoimmunkrankheiten, unter anderem bei Multipler Sklerose, spielt SWAP-70 eine zentrale Rolle. Für Multiple Sklerose gibt es bisher keine therapeutische Heilung.

AUFGABE DER ERFINDUNG

Aufgabe der Erfindung ist es daher ein Verfahren anzugeben, welches es erlaubt gezielt nach Wirkstoffen zu suchen, die als neue Anti-Allergika, sowie zur Behandlung von Osteoporose, Multiple Sklerose und Krebs verwendet werden können und welche insbesondere die Produktion von IgE hemmen. Des Weiteren soll die Erfindung Mittel zur Durchführung des Screeningverfahrens angeben.

BESCHREIBUNG DER ERFINDUNG

Der Erfindung liegt die jüngst gewonnene Erkenntnis zugrunde, dass SWAP-70 dimerisiert, dass die Dimerisierung über eine spezifische, weitgehend einzigartige und eingegrenzte Region des Proteins erfolgt und dass diese Dimerisierung für die Funktion des Proteins (und wahrscheinlich dessen Stabilität) zentral ist. Die Dimerisierungsdomäne wurde als besonders saure Region identifiziert und weist einen extrem hohen Q/E-Gehalt von etwa 41 % auf. Es konnte gezeigt werden, dass die Dimerisierung für die funktionelle F-Aktin Interaktion von SWAP-70, die der Funktion von SWAP-70 zugrunde liegt, benötigt wird. Mutanten, denen die entsprechende Dimerisierungsdomäne fehlt, dimerisieren nicht und bündeln F-Aktinfilamente nicht. Ferner konnte gezeigt werden, dass funktionelle Osteoklasten der Dimerisierungsdomäne von SWAP-70 bedürfen.

Stoffe, die sich an die Dimerisierungsdomäne von SWAP-70 anlagern und damit die Dimerisierung von SWAP-70 verhindern und dadurch die die Aktivität von SWAP-70 inhibieren, eignen sich somit als neue Anti-Allergika, sowie zur Behandlung von Osteoporose und Krebs.

Basierend auf diesen Erkenntnissen stellt die Erfindung ein Screeningverfahren zur Verfügung welches erlaubt neue Wirkstoffe zu identifizieren, welche, durch Anlagerung an die Dimerisierungsdomäne und Inhibition der SWAP-70-Aktivität, die unterstützende Funktion von SWAP-70 in der Tumorgenese, der Tumorzellmigration und -invasion, der knochenabbauenden Osteoklastenaktivität, und der allergischen Reaktion unterbinden.

SWAP-70 ist ein in Domänstruktur und Sequenzanordnung einzigartiges Protein, zu dem es im Gesamtgenom, nur ein einziges verwandtes Protein gibt. Dieses andere Protein trägt jedoch nicht die SWAP-70 typische Dimerisierungssequenz. Daher wird ein Hemmstoff der Dimerisierung von SWAP-70 keine oder kaum Wirkung auf andere Proteine haben und entsprechend spezifisch sein.

Aufgrund seiner Einzigartigkeit stellt SWAP-70 einen idealen zusätzlichen Ansatzpunkt in multifaktorieller Kombinationstherapie wie sie z. B. bei Krebs unabdingbar ist dar. Es wird durch Hemmung von SWAP-70 ein wichtiger zusätzlicher Weg oder Prozess gehemmt. Kombinationstherapie eignet sich ebenso bei Allergien, Autoimmunkrankheiten, insbesondere Multiple Sklerose, und Osteoporose.

SWAP-70 ist meist an ursächlichen Prozessen der o.g. Erkrankungen beteiligt. Hemmung von SWAP-70 wäre also eine ursächliche Therapie statt nur Linderung von Symptomen.

Insbesondere in der IgE-Produktion in B-Zellen agiert SWAP-70 in einzigartiger Weise im Zellkern der Zellen, d.h. eine besonders hohe Spezifität ist gewährleistet.

Die Aufgabe wird gelöst durch die Dimerisierungsdomäne gemäß Anspruch 1 und ein Verfahren zur Identifizierung einer Substanz, welche die Aktivität von SWAP-70 inhibiert gemäß Anspruch 3, wobei das Verfahren folgendes umfasst:

(i) Inkontaktbringen:

(a) mindestens einer Testsubstanz mit

(b) der Dimerisierungsdomäne von SWAP-70

(ii) Detektion des Dimerisierungsgrades von SWAP-70,

(iii) Auswahl einer Testsubstanz, welche die Dimerisierung von SWAP-70 inhibiert.

Die Detektion des Dimersierungsgrades von SWAP-70 kann mit unterschiedlichsten bekannten Methoden erfolgen. Unter Dimerisierungsgrad wird der Anteil der Moleküle verstanden, die als Dimere vorliegen.

In dem erfindungsgemäßen Screening-Verfahren werden Testsubstanzen dazu bevorzugt mit SWAP-70-Monomeren in Kontakt gebracht. Da es sich bei der Dimerisierung um eine reversible Gleichgewichtsreaktion handelt, schließt dies nicht aus, dass ein Teil der SWAP-Moleküle als Dimere vorliegt.

Die Inhibition der Dimerisierung von SWAP-70 kann auf zwei Wegen erfolgen: unmittelbar nach der Translation, also vor der Dimerisierung, oder als Hemmung der re-Dimerisierung von Monomeren. Nach der Dissoziation von Dimeren kann ein Inhibitor die entstehenden Monomere binden und re-Dimerisierung verhindern. Hat der Inhibitor eine sehr hohe Affinität, die höher ist als die Affinität zwischen Monomeren, wird er die Dimerisierung von vornherein unterbinden aber auch effektiv Dimere durch Verhinderung der re-Dimerisierung spalten. Das Gleichgewicht der Reaktion wird zugunsten der Monomere verschoben.

Zur Detektion des Dimerisierungsgrades werden bevorzugt zwei unterschiedlich markierte SWAP-70-Moleküle eingesetzt. Vorzugsweise erfolgt eine Markierung mit Fluoreszenzmarkern oder auch Affinitätstags. Alternativ ist nur eines der SWAP-70-Moleküle markiert und das andere auf einem Träger oder einer Oberfläche fixiert. Alternativ wird Dimerisierung aufgrund durch Dimerisierung veränderter Diffusion in Lösung eines markierten SWAP-70-Moleküls oder zweier unterschiedlicher SWAP-70 Moleküle, oder durch Fluoreszenzkorrelationsspektroskopie (Fluorescene Cross Correlation Spektroskopie - FCCS), gemessen.

Die beiden SWAP-70-Moleküle sind in der Regel in ihrer Sequenz und Struktur identisch und unterscheiden sich nur durch ihre jeweilige Anbindung an einen oder mehrere Marker oder an einen Träger oder eine Oberfläche.

Die Testsubstanzen sind bevorzugt sogenannte "small molecules", d. h. Verbindungen mit einem Molekulargewicht bis 800 Dalton (atomare Masseneinheit u). Die Testsubstanzen sind bevorzugt organische Moleküle oder auch Peptide.

Bei dem erfindungsgemäßen Verfahren kann sowohl ein Test mit einzelnen Testsubstanzen, als auch ein Screening nach mehreren Testsubstanzen parallel erfolgen.

Der SWAP-70-Dimerisierungsgrad bzw. die Interaktion der SWAP-70-Monomere mit den Testsubstanzen wird bevorzugt *in vitro* oder *in situ gemessen.*

*In vitro* (d. h. in zellfreien Verfahren) wird die Wirkung der Testsubstanz(en) auf die SWAP-70-Dimerisierung bzw. die Interaktion zwischen den SWAP-70-Molekülen (nachfolgend zusammen auch als Bindungspartner bezeichnet) bevorzugt unter Benutzung der entsprechenden gereinigten Proteine als Bindungspartner getestet. Dies erfolgt beispielsweise in Bindungsassays, in denen die Assoziation eines markierten mit einem unmarkierten oder zweier verschieden markierter Bindungspartner gemessen werden. Tests dieser Art können u. a. im Multiwell-Verfahren (ähnlich einem ELISA) oder in Lösung (z. B. mittels Alpha-Screen) im high throughput betrieben werden. Marker können z. B. fluoreszierende Farbstoffe sein, die kovalent an einen oder an beide Bindungspartner gebunden werden. Alternativ erfolgt die Markierung radioaktiv, mittels Affinitätstags, Haptameren oder durch Markierung mit fluoreszierenden Proteinen (bevorzugt als Fusionsprotein - z. B. mit GFP). Das Verbleiben der Fluoreszenzsignale am unmarkierten Partner oder die Fluoreszenzinterferenz zwischen assoziierten Partnern wird ausgelesen.

*In situ* (d. h. in Zellen, bevorzugt in Zellkultur) wird die Wirkung der Testsubstanz(en) auf die SWAP-70-Dimerisierung bevorzugt in Zellen gemessen welche SWAP-70 exprimieren. Die Detektion der Interaktion zwischen den Bindungspartnern erfolgt hier bevorzugt ebenfalls per FRET oder auch in Zell-Lysaten. Auch hier erfolgt die Markierung der einzelnen Bindungspartner (SWAP-70-Moleküle) z. B. mittels Affinitäts-Tags (z. B. His-tag) und entsprechenden Affinitätsbindungs-Materialien (wie z. B. $Ni^{2+}$) an diese Materialien gebunden.

Die Wirkung von zellgängigen Testsubstanzen beeinflusst die Bindungspartner ähnlich wie oben bei den *in vitro*-Methoden beschrieben. Bevorzugt werden die *in situ*-Verfahren mit Hilfe von gängigen, leicht kultivierbaren und transfizierbaren Zell-Linien, in denen die Bindungspartner exprimiert werden, durchgeführt. Geeignete Zelllinien sind z. B. NIH3T3, 293T und COS-7. Interaktionen zwischen den Bindungspartnern können vorteilhaft auch in Nicht-Säugerzellsystem analysiert werden, z. B. in Hefezellen (Yeast2 Hybrid-System) oder Insektenzellen (Co-Präzipitation nach Expression), oder nach Co-Translation in zellfreien Systemen (*in vitro* Transkriptions/Translationssysteme, gefolgt von Co-Präzipitationen, sog. pull-downs). Als negative Kontrollen werden bevorzugt entsprechende Zellen oder Zelllinien verwendet in denen die Gene, die für einzelne dieser Proteine codieren, nicht vorhanden sind oder deletiert sind (z. B. Zellen aus SWAP-70-/- Mäusen, wie z. B. embryonale Mausfibroblasten). Multiwell-Formate erlauben high throughput screening Verfahren. Der Begriff Zellen umfasst bevorzugt keine humanen embryonalen Stammzellen, die durch Zerstörung von humanen Embryonen gewonnen wurden.

Bevorzugt wird die Detektion der Interaktion der Bindungspartner *in vitro* oder auch in der Zelle durch Förster-Resonanzenergietransfer (FRET)-Analysen untersucht. Dazu werden die SWAP-70-Moleküle bevorzugt mit zwei zueinander unterschiedlichen Molekülen, die zur Erzeugung eines FRET geeignet sind, markiert. Diese beiden zueinander unterschiedlichen Moleküle werden nachfolgend auch FRET-Donor- und FRET-Akzeptor (oder auch einfach Donor und Akzeptor) genannt.

Der Förster-Resonanzenergietransfer (kurz FRET) ist ein physikalischer Prozess, bei dem Energie eines angeregten Donors strahlungsfrei auf ein Akzeptor im Abstand von etwa 1,5 bis 10 nm übertragen werden kann. Die Moleküle sind dazu so ausgewählt, dass das Emissionsspektrum des Donormoleküls mit dem Anregungsspektrum des Akzeptors überlappt. Sind sowohl Donor als auch Akzeptor Fluoreszenzfarbstoffe, spricht man von einem Fluorescence resonance energy transfer. Ist der Donor oder Akzeptor hingegen eine Chemilumineszenz- oder eine Biolumineszenzquelle wird der Begriff Chemilumineszenz-Resonanzenergietransfer (CRET) bzw. Biolumineszenz-Resonanzenergietransfer (BRET) verwendet.

Für in vitro Assays wird der Donor und/oder der Akzeptor bevorzugt an den Bindungspartner kovalent oder durch Koordination gebunden. Die Markierung des Proteins erfolgt bevorzugt kovalent an freie Aminogruppen oder Carboxylgruppen der Aminosäurenseitenketten (Lysin- oder Aspartat- oder Glutamat-Reste) des Proteins. Bevorzugt werden in der Erfindung als Donoren und Akzeptoren organische oder anorganische Fluoreszenzfarbstoffe verwendet. Bevorzugt sind pro Proteinmolekül 1 bis 40, besonders bevorzugt 1 bis 5 Farbstoffmoleküle gebunden. Als anorganische Fluoreszenzfarbstoffe werden z. B. Europium (bevorzugt $Eu^{3+}$), Cer oder Terbium oder sogenannte Quantum dots (Quantenpunkte), wie z.B. dotierte $LaF_3$ und $LaPO_4$-Nanopartikel, verwendet.

Die Anbindung eines Biomoleküls, wie fluoreszierenden Proteinen (z. B. GFP, YFP und CFP) und/oder Luciferase erfolgt entweder durch Crosslinking oder durch Herstellung eines Fusionsproteins. Fusionsproteine mit derartigen Biomolekülen erlauben vorteilhaft auch die Detektion der Interaktion der Bindungspartner *in situ* (in der Zelle). Lumineszenzquellen (wie Luciferase) und fluoreszierende Proteine können vorteilhaft als Akzeptoren und Donoren eingesetzt werden.

Alternativ zu Zellen können auch multizelluläre Organismen für das Verfahren genutzt werden. Hier wird die Interaktion der Bindungspartner bevorzugt *in vivo* bestimmt. Andere multizelluläre Organismen, sind ausgewählt aus Vertebraten (Mensch ausgenommen) und Invertebraten, wie *Drosophila melanogaster, Caenorhabditis elegans, Xenopus laevis, Oryzias latipes, Danio rerio* oder *Mus musculus,* oder deren Embryonen.

Bevorzugte Donor-Akzeptor-Paare und ihre jeweiligen Anregungsmaxima ($Max_{ex}$) sowie Emissionsmaxima (Maxem) sind ausgewählt aus:

| Donor | | | Akzeptor | | |
|---|---|---|---|---|---|
| Bezeichnung | $Max_{ex}$ | $Max_{em}$ | Bezeichnung | $Max_{ex}$ | $Max_{em}$ |
| Alexa Fluor 488 | 495 nm | 519 nm | Alexa Fluor 555 (oder auch Alexa Fluor 546 oder 568) | 555 nm | 565 nm |
| Alexa Fluor 546 | 556 nm | 573 nm | Alexa Fluor 633 | 632 nm | 647 nm |
| Alexa Fluor 555 | 555 nm | 565 nm | Alexa Fluor 647 | 650 nm | 668 nm |
| Alexa Fluor 568 | 578 nm | 603 nm | Cy5 | 649 nm | 670 nm |
| Alexa Fluor 568 (oder auch Cy3) | 578 nm | 603 nm | Alexa Fluor 633 (oder auch Cy5 oder Alexa Fluor 647) | 632 nm | 647 nm |
| Europium ($Eu^{3+}$) | 395 nm 466 nm | 617 nm | Allophycocyanin | 650 nm | 660 nm |

(fortgesetzt)

| Donor | | | Akzeptor | | |
|---|---|---|---|---|---|
| Bezeichnung | Max$_{ex}$ | Max$_{em}$ | Bezeichnung | Max$_{ex}$ | Max$_{em}$ |
| Eu$^{3+}$ (oder LaF$_3$) | 395 nm | 591 nm | Alexa Fluor 594 (oder auch Alexa Fluor 610 oder 633) | 590 nm | 630 nm |
| LaPO$_4$ (oder Ce oder Tb) | 266 nm | 542 nm | Alexa Fluor 532 | 530 nm | 560 nm |
| zyan fluoreszierendes Protein (CFP) | 452 nm | 505 nm | gelb fluoreszierendes Protein (YFP) | 514 nm | 527 nm |
| Luciferase | | | YFP | 514 nm | 527 nm |
| EGFP | 488 nm | 509 nm | YFP | 514 nm | 527 nm |

FRET-Analysen eignen sich auch für das High throughput screening. An den einen Bindungspartner ist ein FRET-Donor an den anderen ein FRET-Akzeptor gebunden, wobei beiden zusammen ein FRET-Paar bilden. Liegen die beiden Bindungspartner unabhängig voneinander *in vitro* oder in der Zelle tritt kein FRET auf. Durch die Interaktion der beiden Bindungspartner werden die FRET-Donor und FRET-Akzeptor in unmittelbare Nachbarschaft gebracht, wodurch der FRET ausgelöst wird.

Durch den FRET nehmen die Strahlungsemission und die Fluoreszenzlebensdauer des Donors ab, sowie die Akzeptoremission zu. Dies resultiert in einer messbaren Verschiebung der Wellenlänge des emittierten Lichts bei Interaktion der Bindungspartner, welche mit Hilfe geeigneter instrumenteller Methoden nachgewiesen werden. Für diesen Zweck eignen sich beispielsweise Fluoreszenzmikroskope, Flow Cytometer, Fluorimeter oder Plattenspektrophotometer.

Die Testsubstanzen beeinflussen die Interaktion der Bindungspartner. Bevorzugt werden Testsubstanzen ausgewählt, welche die Dimerisierung von SWAP-70 inhibieren bzw. die Interaktion von SWAP-70-Monomeren miteinander verringern.

Die erfindungsgemäße Detektion des Dimerisierungsgrades von SWAP-70 kann ebenfalls indirekt über die Detektion der Molekülgröße mittels Chromatographie erfolgen. Hierzu werden zum einen SWAP-70-Moleküle, zum anderen SWAP-70-Moleküle gemischt mit einer Testsubstanz auf die mobile Phase aufgetragen. SWAP-70-Dimere lassen sich durch unterschiedliche Laufweiten von SWAP-70-Monomeren unterscheiden. Dadurch können Rückschlüsse auf den Einfluss der Testsubstanz auf die Dimerisierung gezogen werden.

Weiterhin kann die Detektion des Dimerisierungsgrades von SWAP-70 mit Hilfe einer Quarzmirkowaage erfolgen. SWAP-70-Moleküle werden dazu auf einem Schwingquarz fixiert. Die Frequenzänderungen unter Zugabe weiterer SWAP-70-Moleküle in Kombination mit einer Testsubstanz werden mit den Frequenzänderungen unter Zugabe weiterer SWAP-70-Moleküle ohne Testsubstanz verglichen. Aus diesem Vergleich lassen sich Schlüsse auf den Dimerisierungsgrad mit und ohne Zugabe der Testsubstanz, und somit auf die Wirkung der Testsubstanz ziehen.

Die erfindungsgemäße Detektion des Dimerisierungsgrades von SWAP-70 kann des Weiteren mittels Surface-Plasmon-Resonanz erfolgen. Hierbei werden SWAP-70-Moleküle an eine Oberfläche gebunden. Gemessen wird die Änderung der Lichtbrechung unter Zugabe von weiteren SWAP-70-Molekülen. Diese Änderung der Lichtbrechung wird mit der Änderung der Lichtbrechung verglichen, die aus einer Zugabe weiterer SWAP-70-Moleküle in Kombination mit einer Testsubstanz resultiert.

Bei Verwendung einer Methode ausgewählt aus Surface-Plasmon-Resonanz, Chromatographie oder Messung der Frequenzänderung mittels einer Quarzmikrowaage müssen sich die SWAP-70-Moleküle nicht zwangsläufig durch ihre jeweilige Anbindung an einen oder mehrere Marker oder an einen Träger oder eine Oberfläche unterscheiden.

Die mit dem erfindungsgemäßen Verfahren ermittelten Testsubstanzen werden bevorzugt weiter auf die biologische Wirksamkeit, insbesondere die Hemmung der IgE Produktion, die Mastzelldegranulierung, die knochenresorbierende Osteoklastenaktivität, die Zelltransformation primärer Zellen zu Tumorzellen, das migratorische Verhalten von Tumorzellen, getestet. Dazu werden bevorzugt B-Lymphozyten in situ oder in vivo zur IgE-Produktion angeregt. Bevorzugt werden dabei isolierte Milzzellen (z. B. der Maus) verwendet, da diese eine große Zahl (> 60 %) an B-Lymphozyten enthalten. Die B-Lymphozyten können, müssen aber dazu nicht aufgereinigt werden. Die B-Lymphozyten können mittels IL-4 (bevorzugt murin und z. B. rekombinant hergestellt) und CD40L (z. B. durch fixierte CD40L-exprimiernde Zellen) zur IgE-Produktion angeregt werden. Die IgE-Produktion kann einfach und mit hohem Durchsatz, z. B. mit Hilfe der FACS Analyse oder ELISA, gemessen werden. Bevorzugt werden Testsubstanzen ausgewählt, welche die IgE Produktion reduzieren.

Die mit dem erfindungsgemäßen Verfahren identifizierten Substanzen eignen sich insbesondere zur Behandlung von IgE-vermittelten Allergien, insbesondere zur Behandlung von IgE abhängigen allergischen Reaktionen bzw. Typ I Allergien und von IgE-abhängigen Autoimmunerkrankungen. Die in erfindungsgemäßen Verfahren identifizierten Substanzen eignen sich insbesondere zur Behandlung von IgE-abhängigen Erkrankungen wie allergischem Asthma, allergischer Rhinitis, allergischer Konjunktivitis, allergischen Reaktionen des Gastrointestinaltrakts (insbesondere Erbrechen und Durchfälle), Neurodermitis, Psoriasis, Kontaktekzem, Urtikaria, allergischen Ödemen, insbesondere dem Larynxödem und dem Angiödem (Quincke-Ödem), anaphylaktischem Schock, allergischer Vaskulitis, Angiitis oder Granulomatose (Churg-Strauss-Syndrom), Hyper-IgE-Syndrom, Omenn-Syndrom, einige Formen des 22q11-Syndroms, der rheumatoiden Arthritis, des Lupus erythematodes, der Typ I Diabetes, des Sjogren's Syndrom, und des bullösen Pemphigoids.

Die mit dem erfindungsgemäßen Verfahren identifizierten Substanzen eignen sich des Weiteren zur Behandlung von Autoimmunkrankheiten wie beispielsweise Multiple Sklerose.

Die mit dem erfindungsgemäßen Verfahren identifizierten Substanzen eignen sich des Weiteren zur Behandlung von Osteoporose durch Hemmung der von SWAP-70 abhängigen knochenresorbierenden Aktivituat der Osteoklasten, sowie Krebs, insbesondere aber nicht beschränkt auf Lymphome, Leukämien, Glioblastome, Krebsstammzellen, Brustkarzinome, Sarkome.

Gegenstand der Erfindung ist auch die Dimerisierungsdomäne von SWAP-70, enthaltend die folgende Sequenz:

QDEETVRKLQARLLEEESSKRAELEKWHLEQQQAIQTTEAEKQELEQQRVMKEQALQEAMAQ

LEQLELERKQALEQYEGVKKKLE (SEQ ID Nr. 1)

oder eine Sequenz mit 70 %, bevorzugt 80 % Sequenzidentität zu der SEQ ID Nr. 1 oder eine Teilsequenz mit mindesten 50, bevorzugt mindestens 70, bevorzugt mindestens 80 Aminosäureresten. Die Dimerisierungsdomäne von SWAP-70 hat eine Länge bis 100 Aminosäureresten.

Die Dimerisierungsdomäne von SWAP-70 zeichnet sich durch einen hohen Anteil an sauren Aminosäurenresten aus. Bevorzugt sind 15 % bis 25 %, vorzugweise 20 % bis 25 % der Aminosäurereste ausgewählt aus Asparaginsäure (Asp/D) und Glutaminsäure (Glu/E), besonders bevorzugt sind 20 % bis 25 % der Aminosäurereste Glutaminsäure (Glu/E).

Die Dimerisierungsdomäne von SWAP-70 enthält zudem einen sehr hohen Anteil an polaren Resten. Bevorzugt sind 50 % bis 70 %, vorzugweise 55 % bis 65 % der Aminosäurereste ausgewählt aus Asparaginsäure (Asp/D), Glutaminsäure (Glu/E), Glutamin (Gln/Q), Lysin (Lys/K), Arginin (Arg/R), Serin (Ser/S) und Threonin (Thr/T). Bevorzugt sind 10 % bis 20 %, vorzugsweise 13 % bis 17 % der Aminosäurereste ausgewählt aus Lysin (Lys/K) und Arginin (Arg/R).

Im erfindungsgemäßen Screening-Verfahren wird die oben beschriebene Domäne verwendet.

Für *in vitro* Assays liegen die SWAP-70-Moleküle jeweils als isolierte Proteine vor.

Die Proteine sind bevorzugt wie oben beschrieben an ein Trägermaterial (z. B. eine Multiwell-Platte oder eine Membran) gebunden oder mit einem Marker versehen. Im Falle, dass ein SWAP-70-Molekül an ein Trägermaterial gebunden ist, erfolgt die Detektion der Dimerisierung bevorzugt ähnlich einem ELISA oder einem ELISPOT-Assay. Dazu wird das zweite SWAP-70-Molekülbevorzugt mit einem Marker (bevorzugt einem Affinititätstags wie Biotin oder einem Haptamer) versehen.

Für *in situ* oder *in vivo* Assays liegen die SWAP-70-Moleküle bevorzugt in einer Zelle oder einem multizellulären Organismus vor.

Die Proteine sind hier bevorzugt wie oben beschrieben ebenfalls mit einem Marker versehen, der bevorzugt ausgewählt ist aus Affinititätstags, Haptameren, fluoreszierenden oder chemiluminizierenden Biomolekülen. Die Markierung erfolgt hier bevorzugt durch Expression als Fusionsprotein (Protein + Marker).

Bevorzugt werden wie oben beschrieben zwei Marker ausgewählt die zur Erzeugung eines Förster-Resonanzenergietransfers (FRET) geeignet sind.

Die Erfindung wird nachfolgend durch Abbildungen und Beispiele näher erläutert ohne auf diese beschränkt zu sein:

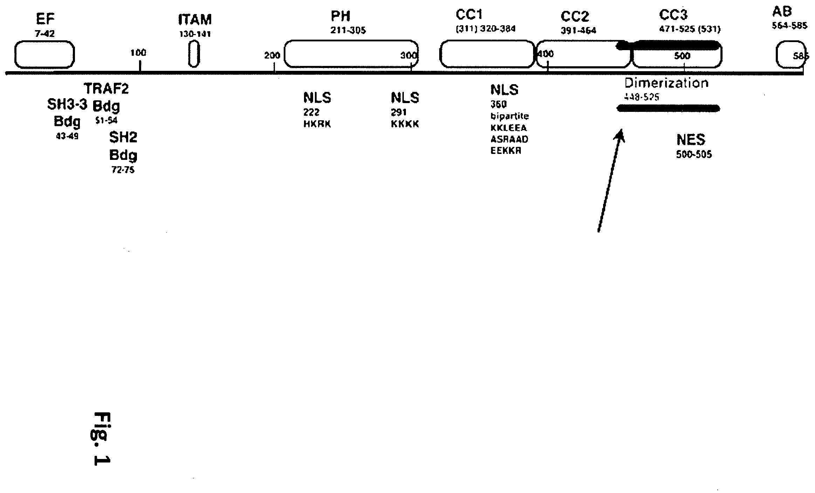

**Fig. 1** zeigt schematisch den Aufbau eines SWAP-70-Proteins. Das SWAP-70-Protein besteht aus der N-terminalen EF-Hand-Domäne, einer Pleckstrin-homologen Domäne (PH), welche PIP$_3$ bindet und für die Lokalisierung der Membran zuständig ist, einer dreigeteilten Coiled-Coil-Domäne (CC1, CC2, CC3) und einer C-terminalen F-Aktin-Bindungsstelle (AB), durch die SWAP-70 spezifisch an nicht-muskuläres Aktin (jedoch nicht an muskuläres Aktin) bindet. Die Dimerisierungsdomäne ist durch einen schwarzen Balken gekennzeichnet und befindet sich überwiegend in der CC3-Domäne.

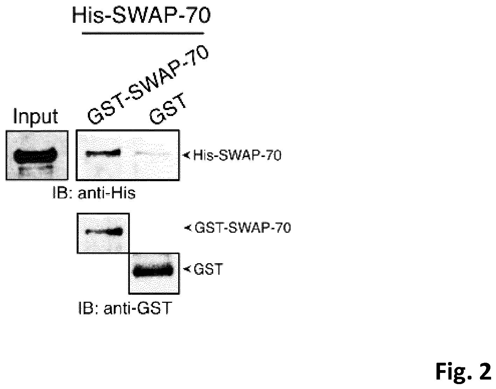

**Fig. 2** zeigt die Ergebnisse der Gelelektrophorese aus Beispiel 1. Es zeigt sich deutlich, dass GST-SWAP-70 und His-SWAP-70 miteinander interagieren. Im oberen Teil der Abbildung sieht man, dass His-SWAP-70 an GST-SWAP-70 gebunden vorliegt, sichtbar gemacht durch Zugabe von anti-His. Ist jedoch nur GST vorhanden, und nicht GST-SWAP-70, so hat His-SWAP-70 keinen Bindungspartner, wird ausgewaschen und bietet keine Anbindungsstelle für anti-His. Im Unteren Teil der Abbildung sind Kontrollen ohne His-SWAP-70 dargestellt.

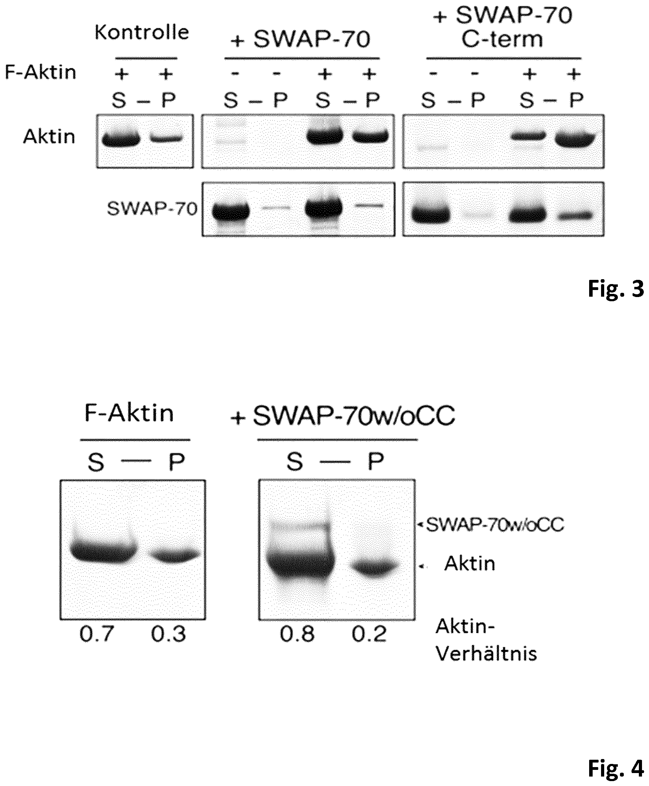

**Fig. 3** zeigt zeigt die Ergebnisse der Gelelektrophorese aus Beispiel 3. S = Überstand, P = Pellet.

**Fig. 4** zeigt zeigt die Ergebnisse der Gelelektrophorese aus Beispiel 4. S = Überstand, P = Pellet.

### Beispiel 1: Pull-down-assay zur Dimerisierung

Ein Pull-down-assay mit aufgereinigtem rekombinierten GST-SWAP-70 und SWAP-70 demonstrierte die Interaktion zwischen diesen beiden Proteinen.

Für den *in vitro* pull-down-assay wurden 0,4 $\mu$M aufgereinigtes GST-SWAP-70 und His-SWAP-70 oder GST zur Kontrolle in 20 mM Tris, pH 7,5, 100 mM NaCl, 10 % Glycerol, 1 mM EDTA, 25 mMCaCl$_2$, 0,1 % NP40 und 1mM DTT gemischt und für 1 h bei 4 °C geschüttelt. Glutathion Agarose Beads wurden anschließend zugegeben und die Proben für 30 Minuten inkubiert, gewaschen und in SDS-Page Ladepuffer resuspendiert. Die Proben wurden anschließend SDS-Page und Immunoblotting unterzogen.

Im Gelbild (Fig. 2) zeigt sich deutlich, dass GST-SWAP-70 und His-SWAP-70 miteinander interagieren.

### Beispiel 2: Gelfiltration zur Dimerisierung

Zur weiteren Untersuchung der Oligomerisierung wurde rekombinantes, aufgereinigtes SWAP-70, SWAP-70w/oCC (Mutante, der die Dimerisierungsdomäne fehlt) und SWAP-70 C-term (verkürztes SWAP-70 mit Aktinbindungsdomäne und einem Teil der Dimerisierungsregion), sowie Protein-Standards (Gelfiltration Marker Kit für Proteine, Molekulargewicht 29-700 kDa, Sigma) einzeln mittels Gelfiltration bei einem Durchfluss von 60 ml/h auf einer Superdex 200 HPLC Säule (Amersham Pharmacia Biotech) analysiert. Die Elutionsvolumen der Protein-Standards wurden in Bezug auf ihr theoretisches Molekulargewicht an eine Kurve angeglichen. Diese Kurve wurde genutzt um die experimentellen Molekulargewichte zu ermitteln.

Die experimentell ermittelte molekulare Masse von SWAP-70 betrug 151 $\pm$ 17,6 kDa, etwa das Doppelte der theoretischen molekularen Masse von SWAP-70 (71,6 kDa), was die Bildung von Dimeren nahelegt.

Die experimentell ermittelte molekulare Masse von SWAP-70 C-term betrug 79 $\pm$ 3,5 kDa, was, verglichen mit der theoretischen molekularen Masse von 19 kDa, eine Tetramerisierung nahelegt.

Die experimentell ermittelte molekulare Masse von SWAP-70w/oCC betrug 50 $\pm$ 3 kDa, was in etwa der theoretischen molekularen Masse von 46 kDa entspricht und zeigt, dass SWAP-70-Mutanten, denen die Dimerisierungsdomäne fehlt, nicht dimerisieren.

Die Ergebnisse sind im Folgenden kurz tabellarisch aufgelistet:

| Protein | Theoretische molekulare Masse [kDa] | Experimentell ermittelte molekulare Masse [kDa] |
|---|---|---|
| SWAP-70 | 71,6 | 151 $\pm$ 17,6 |
| SWAP-70 C-term | 19 | 79 $\pm$ 3,5 |
| SWAP-70w/oCC | 46 | 50 $\pm$ 3 |

### Beispiel 3: SWAP-70 bündelt Aktin-Filamente mittels seiner C-terminalen Region

Die Aktin-Bündelungs-Aktivität von SWAP-70 wurde mittels niedrigtouriger Zentrifugation getestet. Auf gleiche Art wurde die Aktin-Bündelungs-Aktivität von SWAP-70 C-term untersucht.

Zunächst wurde Aktin (3 $\mu$M) polymerisiert. Es folgte eine Vermischung mit SWAP-70, bzw. mit SWAP-70 C-term, Inkubation bei Raumtemperatur für 30 Minuten und Zentrifugation bei 13.000 rpm und 4 °C für 30 Minuten. Eine Kontrolle wurde auf gleiche Art ohne Zugabe von SWAP-70 oder SWAP-70 C-term behandelt. Die Überstände wurden abgenommen und mit eiskaltem Azeton ausgefällt. Die Pellets, welche das quervernetzte F-Aktin enthielten, wurden gewaschen und in 1 X SDS-Page Ladepuffer resuspendiert. Die Proben wurden auf Coomassie-gefärbtem Gel analysiert. Die Proteinbanden wurden mittels Densitometrie unter Benutzung der Fiji Software quantifiziert.

Während in der Kontrolle (linker Teil) deutlich mehr Aktin im Überstand als im Pellet vorhanden war, wurde dieses Verhältnis durch Zugabe von SWAP-70 und SWAP-70 C-term zugunsten des Pellets verschoben. Da SWAP-70 C-term aufgrund von Tetramerisierung in der Lage ist mehr Aktinfilamente zu bündeln als SWAP-70, ist die entsprechende Bande auf dem Gelbild (ganz rechts) stärker als die Aktin-Bande im Pellet bei Zugabe von SWAP-70 (Mitte).

**Beispiel 4: Die Dimerisierung von SWAP-70 führt eine Aktin-Bündelung herbei**

Die Aktin-Bündelungs-Aktivität von SWAP-70w/oCC wurde mittels niedrigtouriger Zentrifugation getestet.

Zunächst wurde Aktin (3 $\mu$M) polymerisiert. Es folgte eine Vermischung mit SWAP-70w/oCC, Inkubation bei Raumtemperatur für 30 Minuten und Zentrifugation bei 13.000 rpm und 4 °C für 30 Minuten. Eine Kontrolle wurde auf gleiche Art ohne der Zugabe von SWAP-70w/oCC behandelt. Die Überstände wurden abgenommen und mit eiskaltem Azeton ausgefällt. Die Pellets, welche das quervernetzte F-Aktin enthielten, wurden gewaschen und in 1 X SDS-Page Ladepuffer resuspendiert. Die Proben wurden auf Coomassie-gefärbtem Gel analysiert. Die Proteinbanden wurden mittels Densitometrie unter Benutzung der Fiji Software quantifiziert.

In der Probe, welche SWAP-70w/oCC enthielt, wurde eine zusätzliche Bande im Vergleich zur Kontrolle detektiert, was zeigt, dass SWAP-70w/oCC nicht an Aktin gebunden hatte. Da SWAP-70w/oCC die Dimerisierungsdomäne fehlt, ist nahegelegt, dass die Aktinbündelung unter Mitwirkung dieser Dimerisierungsdomäne erfolgt.

**Beispiel 5: Detektion der Dimersierung *in vivo* durch FRET**

10 $\mu$g Venus-SWAP-70 und Cerulean-SWAP-70 (Venus ist ein verbessertes Yellow-Fluorescent-Protein und Cerulean ein verbessertes Cyan-Fluorescent-Protein) werden mit Lipofectamin 2000 (Invitrogen) in NIH 3T3-Zellen transfiziert, welche für 16 h auf Poly-(L-Lysin) beschichteten (100 $\mu$g/ml) Deckgläsern angewachsen waren. Den Zellen wird anschließend für einen Zeitraum von 2 Stunden das Serum entzogen, woraufhin sie mit 15 nM EGF (PeproTech) in Dulbecco's Modified Eagle's Medium (DMEM) stimuliert und sofort mit 4% Polyformaldehyd (PFA), PBS (*Phosphate Buffered Saline*) für 10 min fixiert werden. Acceptor Photobleaching FRET Makro, LAS Software (Leica), wird eingesetzt, um Bilder zu erfassen. Die Fluoreszenzintensitäten werden vor und nach dem Bleichen eine Region von Interesse mit einem 510 nm Laser (100% Leistung) erfasst. Die FRET-Effizienz wird mit dem pbFRET v1 plugin (Mike Lorenz, MPI-CBG, Deutschland) auf ImageJ (NIH) oder Fiji bestimmt. Die Proben werden bei Raumtemperatur mit einem Leica TCS SP5 konfokalen Laser-Scanning-Mikroskop (Leica, Deutschland) und eine HCX PL APO 63X 1,4 NA mittels Öl-Objektiv analysiert. Die Bilder werden im linearen Bereich der Reaktion der Detektoren erfasst, wobei eine Pixel Sättigung mit LAS AF Software (Leica) verhindert wird, und mit Fiji analysiert.

**Beispiel 6: Bestimmung der Dimerisierung *in vitro* durch Diffusionsanalyse und Fluoreszenz Cross Correlation Spektroskopie**

Jeweils 1 $\mu$g SWAP-70 Protein werden entweder mit den Fluoreszenfarbstoffen Alexa 488 oder mit Atto 655 markiert, wobei ca. 1 bis 2 Moleküle des jeweiligen Farbstoffs pro Molekül SWAP-70 gebunden werden. Das so markierte SWAP-70 Protein wird in einem confokalen Mikroskop in Lösung in eine Messkammer gebracht und mittels eines (Markierung mit einem Farbstoff) Lasers oder zweier Laser (bei Kombination der zwei unterschiedlich markierten SWAP-70 Moleküle) angeregt. Die Diffusion eines markierten SWAP-70 wird über die Zeit gemessen und korreliert mit dem monomerischen, dimerischen oder multimerischen Zustand des Proteins, der entsprechend daraus abgeleitet werden kann. Die aufgrund der Dimerisierung entstehende wechselseitige Beeinflussung der zwei unterschiedlichen Markierungen wird als Fluoreszenz Cross Correlation bestimmt. Beide Methoden bestätigten die Existenz von SWAP-70 Dimeren. Ein verkürztes (42 kDa basierend auf Gelfiltration und SDS-Gelelektrophorese) Protein, SWAP-70w/oCC, dem die o.g. Dimerisierungssequenz fehlt, erwies sich auch in diesen Untersuchungen als Monomer.

Die folgenden Tabellen zeigen beispielhafte Ergebnisse für Massenkalkulationen aufgrund von Diffusionszeiten. Die Diffusionszeit eines Moleküls ist direkt proportional zu der räumlichen Wurzel der Molekülmasse, das somit näherungsweise nach u.g. Formel bestimmt werden kann. Die Molekülmasse der Farbstoffe wird zur Kalibrierung herangezogen.

$$\frac{\tau_d^{S70}}{\tau_d^{dye}} = \frac{\sqrt[3]{MW^{S70}}}{\sqrt[3]{MW^{dye}}}$$

Die gemessenen Diffusionszeiten der Farbstoffe Atto 655 und Alexa488 wurden gemäss dieser Formel genutzt, um die Molekülmasse von SWAP-70 oder des o.g. SWAP-70w/oCC Fragments zu berechnen. Tabelle 1 zeigt die Diffusionszeiten.

Tabelle 1. Diffusionszeit

| Diffundierendes Molekül | SWAP-70 rot (Alexa) | SWAP-70 grün (Atto) | Atto 655 | Alexa 488 | 42 kDa Fragment red | 42 kDa fragment green |
|---|---|---|---|---|---|---|
| Diffusionszeit (μs) | 247±5 | 207±7 | 37±1.4 | 33±0.6 | 161±3 | 130±2 |

Tabelle 2 zeigt die sich aus der von der Diffusionszeit abgeleiteten Molekülmasse ergebende Anzahl Monomere pro Molekül für entweder rot oder grün gefärbtes SWAP-70 basierend auf den als Standards verwendeten einzelnen Farbstoffen oder dem SWAP-70w/oCC Fragment.. Die Resultate zeigen eine sehr gute Approximation an eine dimerische Molekülstruktur und bestätigen somit die o.g. dimeres SWAP-70 zeigenden Ergebnisse der Gelfiltration.

Tabelle 2. Anzahl von SWAP-70 Monomeren per Molekül SWAP-70, basierend auf den angegebenen Standards.

| Standard | N of monomers SWAP-70 red | N of monomers SWAP-70 green |
|---|---|---|
| 42 kDa fragment | 2.1±0.3 | 2.6±0.5 |
| Atto 655 or Alexa 488 | 2.6±0.6 | 2.3±0.4 |

**Patentansprüche**

1. Dimerisierungsdomäne von SWAP-70 mit einer Länge bis 100 Aminosäureresten enthaltend die Sequenz

   QDEETVRKLQARLLEEESSKRAELEKWHLEQQQAIQTTEAEKQELEQQRVMKEQALQEA

   MAQLEQLELERKQALEQYEGVKKKLE (SEQ ID Nr. 1)

   oder eine Sequenz mit 70 %, bevorzugt 80 % Sequenzidentität zu der SEQ ID Nr. 1 oder eine Teilsequenz mit mindesten 50, bevorzugt mindestens 70, bevorzugt mindestens 80 Aminosäureresten.

2. Verwendung einer Dimerisierungsdomäne nach Anspruch 1 zur Identifizierung einer Substanz, welche die Aktivität von SWAP-70 inhibiert.

3. Verfahren zur Identifizierung einer Substanz, welche die Aktivität von SWAP-70 inhibiert, wobei das Verfahren folgendes umfasst:

   (i) Inkontaktbringen:

   (a) einer Testsubstanz mit
   (b) der Dimerisierungsdomäne von SWAP-70 gemäß Anspruch 1,

   (ii) Detektion des Dimerisierungsgrades von SWAP-70,
   (iii) Auswahl einer Testsubstanz, welche die Dimerisierung von SWAP-70 inhibiert.

4. Verfahren nach Anspruch 3, **dadurch gekennzeichnet, dass** mit Fluoreszenzmarkern markierte SWAP-70-Moleküle verwendet werden.

5. Verfahren nach Anspruch 3 oder 4, **dadurch gekennzeichnet, dass** in Schritt (ii) ein FRETbasierter Assay durchgeführt wird.

6. Verfahren nach Anspruch 3, **dadurch gekennzeichnet, dass** Schritt (ii) mit einer Methode ausgewählt aus Surface-Plasmon-Resonanz, Fluoreszenzkorrelationsspektroskopie, Chromatographie oder mit einer Quarzmikrowaage durchgeführt wird.

**7.** Verfahren nach einem der Ansprüche 3 bis 6, **dadurch gekennzeichnet, dass** die Interaktion der unterschiedlich markierten SWAP-70-Moleküle *in vitro* oder *in situ* gemessen wird.

**Claims**

**1.** A dimerisation domain of SWAP-70 with a length up to 100 amino acid residues containing the sequence

```
QDEETVRKLQARLLEEESSKRAELEKWHLEQQQAIQTTEAEKQELEQQRVMKEQ
ALQEAMAQLEQLELERKQALEQYEGVKKKLE (SEQ ID no. 1)
```

or a sequence with 70 %, preferably 80 % sequence identity with SEQ ID no. 1 or a partial sequence with at least 50, preferably at least 70, preferably at least 80 amino acid residues.

**2.** Use of a dimerisation domain according to claim 1 for identifying a substance which inhibits the activity of SWAP-70.

**3.** A method for identifying a substance which inhibits the activity of SWAP-70, wherein the method comprises the following steps:

(i) bringing into contact:

(a) a test substance with
(b) the dimerisation domain of SWAP-70 according to claim 1,

(ii) detecting the degree of dimerisation of SWAP-70,
(iii) selecting a test substance which inhibits the dimerisation of SWAP-70.

**4.** The method according to claim 3, **characterised in that** SWAP-70 molecules labelled with fluorescent markers are used.

**5.** The method according to claim 3 or 4, **characterised in that** in step (ii) a FRET-based assay is performed.

**6.** The method according to claim 3, **characterised in that** step (ii) is performed with a method selected from surface plasmon resonance, fluorescence correlation spectroscopy, chromatography or with a quartz crystal microbalance.

**7.** The method according to any one of claims 3 to 6, **characterised in that** the interaction of the differently labelled SWAP-70 molecules is measured *in vitro* or *in situ.*

**Revendications**

**1.** Domaine de dimérisation du SWAP70 d'une longueur jusqu'à 100 résidus d'acides aminés, contenant la séquence :

```
QDEETVRKLQARLLEEESSKRAELEKWHLEQQQAIQTTEAEKQELEQQRVMKEQ
ALQEA MAQLEQLELERKQALEQYEGVKKKLE (SEQ ID n°1)
```

ou une séquence avec une identité de séquence de 70 %, de préférence de 80 % avec la SEQ ID n°1 ou une séquence partielle avec au moins 50, de préférence au moins 70, de manière préférentielle, au moins 80 résidus d'acides aminés.

**2.** Utilisation d'un domaine de dimérisation selon la revendication 1 pour l'identification d'une substance, laquelle inhibe l'activité du SWAP70.

**3.** Procédé destiné à identifier une substance, laquelle inhibe l'activité du SWAP-70, le procédé comprenant :

(i) la mise en contant :

(a) d'une substance d'essai avec
(b) le domaine de dimérisation du SWAP70 selon la revendication 1,

(ii) la détection du taux de dimérisation du SWAP70,
(iii) le choix d'une substance d'essai, laquelle inhibe la dimérisation du SWAP70.

4. Procédé selon la revendication 3, **caractérisé en ce qu'**on utilise des molécules du SWAP70 marquées par marqueurs fluorescents.

5. Procédé selon la revendication 3 ou 4, **caractérisé en ce que** dans l'étape (ii), on procède à un essai basé sur le FRET.

6. Procédé selon la revendication 3, **caractérisé en ce qu'**on procède à l'étape (ii) avec une méthode choisie parmi la résonance plasmatique de surface, la spectroscopie de corrélation par fluorescence, la chromatographie ou à l'aide d'une microbalance à quartz.

7. Procédé selon l'une quelconque des revendications 3 à 6, **caractérisé en ce qu'**on mesure *in vitro* ou *in situ* l'interaction entre les molécules de SWAP70 différemment marquées.

Fig. 1

His-SWAP-70

Input GST-SWAP-70 GST

◄His-SWAP-70

IB: anti-His

◄GST-SWAP-70

◄GST

IB: anti-GST

**Fig. 2**

EP 2 954 321 B1

**Fig. 3**

**Fig. 4**

**IN DER BESCHREIBUNG AUFGEFÜHRTE DOKUMENTE**

**In der Beschreibung aufgeführte Patentdokumente**

- US 6528284 B1
- US 2003143611 A1
- WO 9903991 A1
- US 20030166018 A1
- WO 03074652 A2
- WO 2011141267 A1
- DE 102013202206

SEQUENCE LISTING

<110> Technische Universität Dresden

<120> Methode zur Inhibierung des Proteins SWAP-70

<130> 00017P0215DEWO

<150> DE 10 2013 202 206.9
<151> 2013-02-11

<160> 1

<170> PatentIn version 3.5

<210> 1
<211> 85
<212> PRT
<213> Homo sapiens

<400> 1

```
Gln Asp Glu Glu Thr Val Arg Lys Leu Gln Ala Arg Leu Leu Glu Glu
1               5               10              15

Glu Ser Ser Lys Arg Ala Glu Leu Glu Lys Trp His Leu Glu Gln Gln
        20              25              30

Gln Ala Ile Gln Thr Thr Glu Ala Glu Lys Gln Glu Leu Glu Gln Gln
        35              40              45

Arg Val Met Lys Glu Gln Ala Leu Gln Glu Ala Met Ala Gln Leu Glu
    50              55              60

Gln Leu Glu Leu Glu Arg Lys Gln Ala Leu Glu Gln Tyr Glu Gly Val
65              70              75              80

Lys Lys Lys Leu Glu
            85
```